# EUROPEAN PATENT SPECIFICATION

(11) **EP 0 459 536 B1**
(45) Date of publication and mention of the grant of the patent: **04.09.1996**
(21) Application number: 91113550.7
(22) Date of filing: 27.11.1987
(51) Int. Cl.: C07D 219/06, C07D 221/20, C07F 9/64, C07H 17/02, C12Q 1/34, C12Q 1/40

(54) **Chromogenic acridinone enzyme substrates**
Acridinone als chromogene Enzymsubstrate
Acridinones comme substrats d'enzyme chromogènes

(30) Priority: 09.12.1986 US 939855
(43) Date of publication of application: 04.12.1991
(62) Divisional of application: 87117548.5
(73) Proprietor: Bayer Corporation, Pittsburgh, PA 15219-2502 (US)
(72) Inventor: Corey, Paul F., Elkhart, IN 46516 (US)
(74) Representative: Dänner, Klaus, Dr.

(56) References cited:
- NEW PHYTOLOGIST vol. 77, no. 1, 1976, pages 1 - 9; R. HILL ET AL: "Uncoupling of electron transport by anionic quinonoid redox indicator dyes"

## Description

This is a divisional of EP 270 946 which relates to chromogenic compunds which are useful as optical indicator compounds in analytical test systems. The present invention relates to novel chromogenic acridinone compounds and their use in preparation of the indicators of EP 270 946.

Hill et al. in New Phythol., 1976, Vol. 77, pages 1 - 9, described quinonoid redox indicator dyes and a process of making them. The same applies to Kehrman et al. (Helv. Chim.Acta, Vol. 8, 1925, pages 23-27) and Donovan et al. (Journal of the Chemical Society, Part C, 1970, pages 2462 - 2466).

Accordingly, it is an object of the present invention to provide chromogenic acridinone compounds which can be employed as intermediate compounds in the preparation of the compunds of EP 270 946.

### SUMMARY OF THE INVENTION

The present invention provides novel 7-hydroxy-1,3-dihalo-9,9-dimethyl-9H-acridin-2-one chromogen intermediates which are particularly useful for preparation of the chromogenic acridinone enzyme substrate compounds of the present invention and methods of preparation thereof.

### BRIEF DESCRIPTION OF THE DRAWINGS

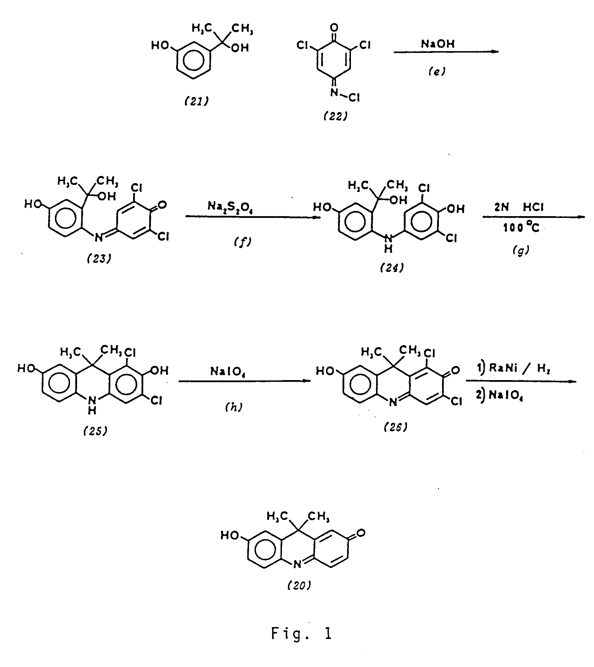

Fig. 1 is a flow diagram of the synthetic pathway for the preparation of the 7-hydroxy-9,9-dimethyl-9H-acridin-2-one chromogens employing the 7-hydroxy-1,3-dichloro-9,9-dimethyl-9H-acridin-2-one intermediate.

### DESCRIPTION OF THE PREFERRED EMBODIMENTS

Accoridng to the present invention, novel 7-hydroxy-1,3-dihalo-9H-acridin-2-one derivatives of the tautomeric formula (19): 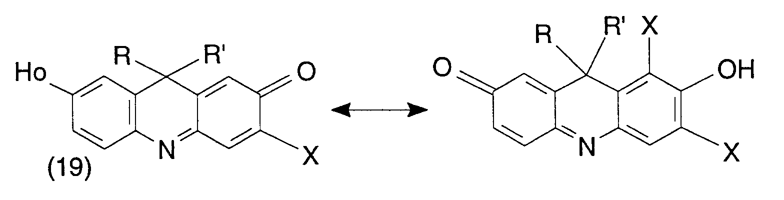 are provided where R and R', which can be the same or different, are alkyl or aryl as heretofore described, preferably lower alkyl or phenyl, and X is a halo radical, preferably bromo or chloro. Such intermediates are particularly useful for the preparation of a dimethyl acridinone chromogen (20) (Fig. 1) which can then be reacted with a radical Y of the Y-OH compound to provide the chromogenic enzyme substrate compounds of the present invention.

In particular, as illustrated in Fig. 1, the dimethyl acridinone chromogen (20) can be prepared by first reacting 3-(1-hydroxy-1-methylethyl)phenyl (21) (Bruce, et al., J. Chem. Soc, (C), 1627 (1966)) and 2,6-dichloroquinone-4-chloroimide (22) (Gibbs, et al., U.S. Public Health Reports, Supp. 69 and Chem. Abs., Vol. 23, 3450 (1929)) with sodium hydroxide in aqueous tetrahydrofuran (reaction (e)) under appropriate conditions to result in the dichloro-substituted indophenyl (23), which is then reduced (reaction (f)) to result in compound (24), acid cyclized (reaciton (g)) to result in compound (25), and then oxidized (reaction (h)) to result in the novel 7-hydroxy-1,3-dichloro-9,9-dimethyl-9H-acridin-2-one (26) of the present invention. The dichloro-derivative (26) is first treated with (1) Raney Nickel and hydrogenated, and then treated with (2) sodium periodate to result in the dimethyl acridinone chromogen (20).

### Example 1

### Synthesis of 7-Hydroxy-9,9-diemthyl-9H-acridin-2-one (20) From 7-Hydroxy-1,3-dichloro-9,9-dimethyl-9H-acridin-2-one Intermediate (26) (Fig. 1).

### (a) Synthesis of 7-Hydroxy-1,3-dichloro-9,9-dimethyl-9H-acridin-2-one (26)

A mixture of 2-(3'-hydroxyphenyl)-2-propanol (*21*) (1.52 g; 10.0 mmol) prepared as described by Bruce, *et al*., *J*. *Chem*. *Soc*. *(C)*, 1627 (1966) and 2,6-dichloroquinone-4-chloroimide (*22*) (Aldrich Chemical Co., Milwaukee, WI, USA) (2.10 g; 10.0 mmol) in tetrahydrofuran (5 mL) was diluted with H₂O (5 mL), chilled in an ice bath and dropwise treated, over 10 min, with aqueous 2M NaOH (10.5 mL; 21 mmol). The resulting deep blue reaction mixture was allowed to stir at 0°C for 1.5 hours and was then blended into a vigorously agitated mixture of saturated aqueous NH₄Cl (500 mL) and ethyl acetate (300 mL). The phases were separated and the aqueous phase was extracted once with ethyl acetate (100 mL), then the combined ethyl acetate layers were washed once with saturated aqueous NH₄Cl (200 mL). The resulting ethyl acetate solution of (*23*) was then washed twice with a solution of Na₂S₂O₄ (25 g) in H₂O (250 mL). The combined aqueous washes were extracted once with ethyl acetate (50 mL) then the combined ethyl acetate layers were washed once with saturated aqueous NaCl (brine) (150 mL), dried over Na₂SO₄, filtered and freed of solvent *in vacuo* to obtain a crude form of (*24*) as a brown tar which was used without purification. A solution of the crude (*24*) in methanol (10 mL) was slowly blended into rapidly stirring, deoxygenated (by inert gas purge) aqueous 2M HCl (250 mL) at ambient temperature. The resulting suspension was heated in a 100°C oil bath under an inert gas atmosphere for 1.25 hours during which time a dark, gummy solid separated. The reaction mixture was cooled to ambient temperature, stirred rapidly with ethyl acetate (200 mL) and the phases separated; the aqueous layer was extracted once with ethyl acetate (200 mL) and then the combined ethyl acetate layers were washed once with brine (50 mL). The resulting ethyl acetate solution of (*25*) was oxidized by stirring vigorously for 15 minutes with a solution of NaIO₄ (3g) in H₂O (100 mL), then the phases were separated and the ethyl acetate layer was washed once with brine (100 mL), dried over Na₂SO₄, filtered and evaporated to dryness *in vacuo* to obtain a crude form of 7-hydroxy-1,3-dichloro-9,9-dimethyl-9H-acridin-2-one (*26* which was then taken up in boiling ethyl alcohol (500 mL) and concentrated by boiling to a volume of *ca*. 150 mL. Upon cooling, the compound (*26*) separated in the form of fine ruddy-black needles to obtain, after two crops, 2.5 g (82%). A portion of the first crop was recrystallized from ethyl alcohol to obtain an analytical sample of (*26*) having no mp <250°C.
IR(KBr) cm⁻¹ 1624, 1492, 1451, 1304, 985, 500;
¹H NMR (DMSO-d⁶) δ 1.74 (s, 6H), 6.84 (d of d, J₁=8.5 Hz and J₂=2.4 Hz, 1H), 7.06 (d, J=2.4 Hz, 1H),
7.50 (d, J=8.5 Hz, 1H), 7.76 (s, 1H);
¹³C NMR (DMSO-d⁶) ppm 172.07, 162,70, 145.73, 141.18, 140,85, 139.23, 134.8, 134.6, 134.28, 132.46, 115.95, 114.12, 26.27 (1 coincident band, one hidden by solvent bands);

| Anal. Calcd. for C₁₅H₁₁Cl₂NO₂: | | | |
|---|---|---|---|
| | C, 58.46; | H, 3.60; | N, 4.55 |
| Found: | C, 58.71; | H, 3.83; | N, 4.34. |

### (b) Synthesis of 7-Hydroxy-9,9-dimethyl-9H-acridin-2-one (20)

A solution of the dichloro compound (*26*) from step(a) of the present example (2.0 g; 6.49 mmol) in aqueous 1M NaOH (60 mL) and ethyl alcohol (40 mL) was treated with Raney Nickel 2800 (W.R. Grace and Co., Baltimore, MD, USA) (1 teaspoon) and hydrogenated at 40 psi H₂ with agitation at *ca*. 70°C for 5 hours. After cooling to ambient temperature, the reaction mixture was filtered through diatomaceous earth (Celite®, Johns-Manville Corp. Denver, CO, USA), diluted with H₂O (500 mL) then acidified with aqueous 2M HCl (100 mL) and extracted five times with ethyl acetate (200 mL @). The combined ethyl acetate extracts were vigorously stirred with a solution of NaIO₄ (4 g) in H₂O (300 mL) for 10 minutes at ambient temperature then treated with aqueous 1M HCl (109 mL). The phases were separated and the aqueous layer was washed twice with ethyl acetate (50 mL @); the combined ethyl acetate layers were washed once with brine (50 mL), dried over MgSO₄, filtered and evaporated to dryness *in vacuo*. Traces of residual water were removed by azeotroping with toluene and then vacuum drying at 60°C to obtain the dimethyl chromogen (*20*) (1.38 g; 89%) in the form of a red powder. Recrystallization from ethyl acetate/hexane (1:1) afforded the dimethyl chromogen (*20*) in the form of ruby-red needles identical to that prepared by the method of Hill, *et al*.*, supra*.

## Claims

1. An acridinone chromogen characterized by the tautomeric formula 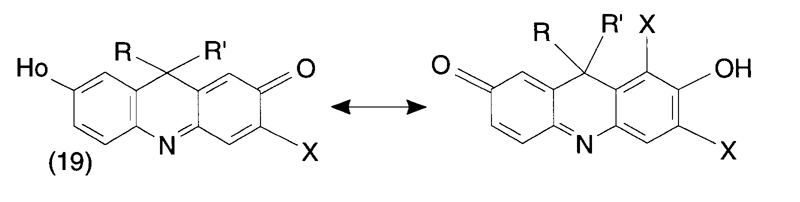 wherein R and R', which can be the same or different, are C₁-C₆ alkyl, phenyl or naphtyl, and X is halo.

2. The chromogen of claim 1 wherein R and R', which can be the same or different, are alkyl containing from 1 to 6 carbon atoms or phenyl and X is bromo or chloro.

## Patentansprüche

1. Acridinonchromogen, **gekennzeichnet** durch die tautomere Formel 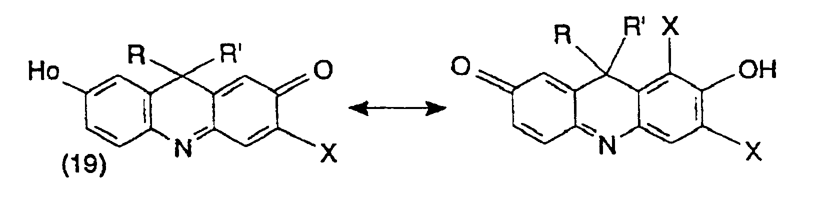 worin R und R', die gleich oder verschieden sein können, Alkyl, Phenyl oder Naphthyl sind, und X ist Halogen.

2. Chromogen nach Anspruch 1, worin R und R', die gleich oder verschieden sein können, Alkyl mit 1 bis 6 Kohlenstoffatomen oder Phenyl sind, und X ist Brom oder Chlor.

## Revendications

1. Chromogène d'acridinone caractérisé par la formule tautomère 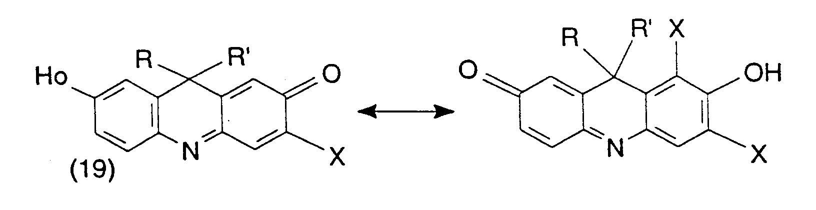 dans laquelle R et R', qui peuvent être identiques ou différents, sont alcoyles, phényles ou naphtyles et X est halogène.

2. Chromogène selon la revendication 1 dans lequel R et R', qui peuvent être identiques ou différents, sont alcoyles contenant 1 à 6 atomes de carbone ou phényles et X est le brome ou le chlore.
